(19) Europäisches Patentamt

European Patent Office

Office européen des brevets

(11) **EP 0 699 083 B1**

(12) **FASCICULE DE BREVET EUROPEEN**

(45) Date de publication et mention
de la délivrance du brevet:
**27.10.1999 Bulletin 1999/43**

(21) Numéro de dépôt: **95910593.3**

(22) Date de dépôt: **23.02.1995**

(51) Int. Cl.$^6$: **A61M 13/00**

(86) Numéro de dépôt international:
**PCT/FR95/00218**

(87) Numéro de publication internationale:
**WO 95/23006 (31.08.1995 Gazette 1995/37)**

(54) **INSUFFLATEUR MEDICAL A REGULATION AUTOMATIQUE DE DEBIT GAZEUX**

MEDIZINISCHER GASINHALATOR MIT AUTOMATISCHER GASFLUSSREGELUNG

MEDICAL GAS INSUFFLATOR WITH AUTOMATIC GAS FLOW CONTROL

(84) Etats contractants désignés:
**AT BE CH DE ES FR GB IT LI**

(30) Priorité: **25.02.1994 FR 9402483**

(43) Date de publication de la demande:
**06.03.1996 Bulletin 1996/10**

(73) Titulaire: **Ognier, Jean-François**
**84500 Bollene Saint Pierre (FR)**

(72) Inventeur: **Ognier, Jean-François**
**84500 Bollene Saint Pierre (FR)**

(74) Mandataire: **Bratel, Gérard**
**Cabinet GERMAIN & MAUREAU,**
**12, rue Boileau,**
**BP 6153**
**69466 Lyon Cedex 06 (FR)**

(56) Documents cités:
**EP-A- 0 293 669        EP-A- 0 517 190**
**EP-A- 0 569 241        US-A- 5 006 109**

**Description**

La présente invention concerne un insufflateur médical à régulation automatique de débit gazeux, utilisé dans le domaine de l'endoscopie diagnostique et chirurgicale pour créer à l'intérieur du corps humain ou animal, par insufflation d'un gaz neutre, une cavité d'observation et/ou un espace opératoire.

L'évolution de l'endoscopie diagnostique vers l'endoscopie chirurgicale a induit de nouvelles contraintes auxquelles doivent satisfaire les appareils et instruments mis en oeuvre. Les insufflateurs médicaux appartiennent à cette catégorie de matériels.

D'abord conçus pour dilater la cavité siège de l'observation puis, plus récemment, pour créer un "espace opératoire aseptique", leurs caractéristiques ont dû s'adapter aux moyens thérapeutiques mis en oeuvre par les chirurgiens endoscopistes, moyens qui sont de nature à contrarier leur bon fonctionnement (aspirateurs, vaporisateurs, gaz de refroidissement des fibres et guides de faisceaux laser, appareils d'électro-chirurgie sous gaz neutre, etc...) ou qui requièrent de plus grands débits pour permettre une ventilation de la cavité opératoire (ventilation des fumées d'électro-chirurgie ou des lasers...).

A la demande des chirurgiens, cette évolution s'est toujours traduite par une augmentation des débits instantanés, et par voie de conséquence, des pressions d'insufflation qui atteignent couramment des valeurs de 10500 à 13000 Pa (80 à 100 mm Hg), voire davantage. De ce fait, les appareils actuellement disponibles sur le marché présentent des seuils de sécurité (seuils fixes limitant la pression d'insufflation) qui ne sont plus compatibles avec les conditions requises pour la sécurité des patients. Ces appareils sont tous du type "régulateurs de pression". En fonctionnement normal, afin de compenser de faibles fuites, ils réalisent des cycles d'insufflation très courts sous débits et pressions d'insufflation maximaux.

L'observation montre que pendant près de 90 % de la durée des interventions, le débit utile reste inférieur à 3 l/min alors que dans les conditions extrêmes, les débits instantanés peuvent atteindre 12 à 15 l/min.

De façon générale, les paramètres requis ou à respecter sont les suivants :

-    maintenir une pression intracavitaire comprise entre 0 et 3300 Pa (0 et 25 mm Hg) ;
-    limiter la pression d'insufflation au niveau distal de l'aiguille d'insufflation ou aiguille de Veress en deçà d'un seuil de sécurité (à la connaissance du Déposant, il n'y a jamais eu d'expérimentation clinique réalisée en vue de déterminer ce paramètre ; il semble néanmoins indispensable de rester en deçà de la pression artérielle locale soit 10500 à 13000 Pa, soit 80 à 120 mm Hg) ;
-    ne mettre en oeuvre qu'un seul tube de liaison et un seul dispositif trans-péritonéal (aiguilles ou trocard) pour réaliser l'arrivée du gaz, la mesure de la pression intracavitaire et l'évacuation des fumées ou des surpressions intracavitaires ;
-    atteindre voire dépasser des débits instantanés de gaz insufflé de 12 litres/minute.

Différents dispositifs d'insufflation gazeuse automatique, de mesure de la pression d'insufflation et de mesure de la pression intracavitaire ont déjà été envisagés. Les systèmes proposés combinent généralement un dispositif de mesure, des moyens de détente du gaz insufflé comportant un ou plusieurs réservoirs intermédiaires, et une régulation automatique du débit gazeux.

Le document FR-A-2 303 512 (WIEST), ou son équivalent US-A-3 982 533, décrit un dispositif d'introduction dans la cavité abdominale d'une quantité limitée de gaz carbonique $CO_2$ sous pression contenu dans un réservoir, utilisant une voie de contrôle commandant l'insufflation et mesurant la pression d'insufflation, et une voie de mesure de la pression statique ou pression intracavitaire. Le dispositif nécessite deux aiguilles de Veress d'insufflation ou une aiguille de Veress à deux voies.

Le document DE-A-25 44 467 (WOLF Richard GmbH) décrit un dispositif d'introduction dans la cavité abdominale de gaz carbonique $CO_2$ contenu dans un réservoir sous pression utilisant un détendeur, une voie d'insufflation contrôlée par une électrovanne, et une voie de mesure commandant l'ouverture de ladite électrovanne. Ce dispositif nécessite aussi deux aiguilles de Veress d'insufflation ou une aiguille de Veress à deux voies.

Le document DE-A-28 03 646 (SEMM Kurt) concerne un dispositif d'introduction dans la cavité abdominale de gaz carbonique $CO_2$, utilisant un ensemble multi-étagé comportant plusieurs détendeurs et réservoirs intermédiaires, caractérisé par un système de dérivation multivoies de contrôle de débit. Tel que réalisé, ce dispositif est complété par d'autres aménagements, décrits dans les documents suivants.

Ainsi, le document DE-A-30 00 218 (SEMM Kurt) a pour objet un dispositif de mesure et de contrôle statique de la pression intracavitaire ne nécessitant qu'une seule aiguille et qu'un seul tube reposant sur un cycle de fonctionnement en deux temps, à savoir un temps d'insufflation durant lequel l'appareil mesure la pression d'insufflation (pression dynamique), et un temps de repos durant lequel l'appareil mesure la pression intracavitaire (pression statique).

En complément, le document DE-A-34 13 631 (SEMM Kurt) révèle un dispositif de calcul de la pression intracavitaire à partir de la vitesse d'écoulement, de la résistance à l'écoulement et de la pression d'insufflation.

Dans la pratique courante, les équipes chirurgicales ont opté pour les appareils ne nécessitant qu'une voie d'insufflation (tube de liaison et aiguille de Veress) assurant alternativement la mesure des pressions d'insufflation et intracavitaire, rendant obsolètes les appareils à deux voies. L'inconvénient principal des moyens mis en oeuvre à ce jour réside dans l'impossibilité de contrôler la pression intracavitaire lors de la phase d'insufflation, donc l'impossibilité de réagir à toute modification des paramètres au niveau de la cavité opératoire (surpression accidentelle, fuite importante, réveil du patient...).

Deux autres défauts sont plus généralement reprochés aux insufflateurs du marché :

- ils ne comportent pas de dispositif de décharge en cas de surpression intracavitaire ;
- l'injection de gaz froid provoque des troubles métaboliques, amplifiés par l'anesthésie.

La présente invention vise à éviter ces inconvénients, et à cet effet elle a pour but de fournir un insufflateur permettant :

- de réaliser et maintenir une pression intracavitaire de référence, dite "pression de consigne", par régulation du débit d'insufflation de gaz avec compensation stricte des fuites hors de la cavité opératoire ;
- de déterminer la pression intracavitaire à partir de la pression d'insufflation et de l'évaluation des pertes de charge ;
- de réaliser une décharge non polluante en cas de surpression intracavitaire ;
- de moduler la pression maximale d'insufflation (seuil de sécurité) en fonction du débit instantané ;
- de réchauffer le gaz insufflé à la température du corps.

A la base de l'invention, il y a la constatation (permise par les moyens de mesure de grande précision aujourd'hui disponibles, tels que capteurs électroniques et oscilloscopes) que pour un débit de gaz donné, l'écart entre d'une part la pression d'insufflation Pi, mesurée en tête du circuit d'insufflation avec tube de liaison et aiguille de Veress, et d'autre part la pression intracavitaire Pa, est constant. Cet écart, variable suivant le débit et la configuration du circuit d'insufflation, correspond à la perte de charge du circuit ci-après dénommée $\Delta$p.

Au repos, cette perte de charge est nulle et la pression Pi mesurée en tête du circuit d'insufflation est égale à la pression intracavitaire Pa.

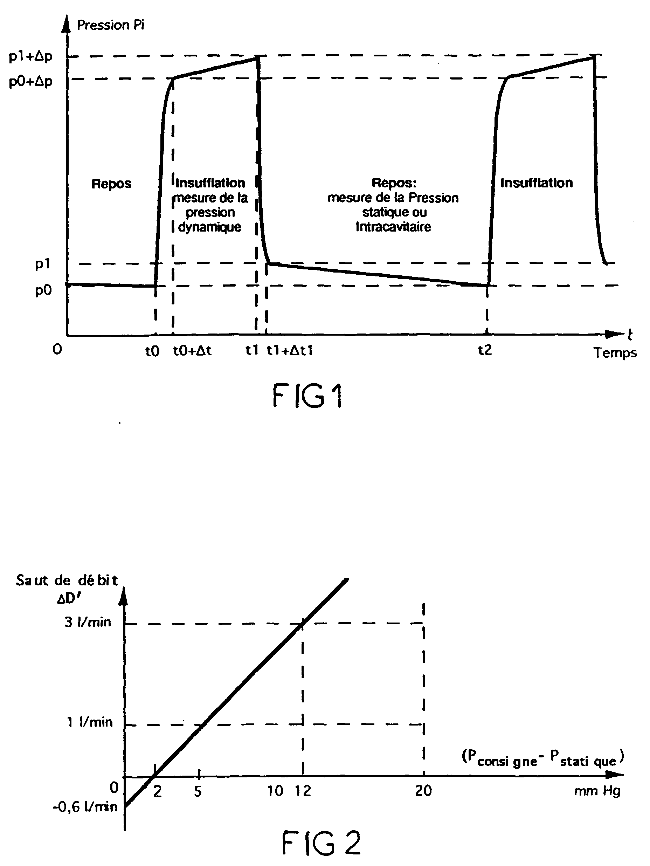

En début d'insufflation, la pression Pi mesurée en tête du circuit passe très rapidement de la valeur de la pression intracavitaire à celle de la pression d'insufflation, comme l'illustre la courbe de variation de la pression d'insufflation selon la figure 1 du dessin annexé. D'après les expériences effectuées par le Déposant, cette phase transitoire (entre les instants t0 et t0 + $\Delta$t) dure de 80 à 100 millisecondes.

Durant cette phase transitoire, et compte tenu de sa brièveté, la variation de la pression intracavitaire est insignifiante. On peut en déduire que la perte de charge $\Delta$p(t0) du circuit à l'instant t0 et pour le débit D(t0) est égale à la variation de la pression d'insufflation Pi durant cette phase transitoire.

Durant tout ce cycle d'insufflation, on peut suivre la progression de la pression intracavitaire en effectuant le calcul suivant :

$$P \text{ intracavitaire} = P \text{ insufflation} - \Delta p$$

les différentes phases du cycle pouvant être décrites comme suit :

- de 0 à t0 : l'insufflateur est au repos. La pression intracavitaire décroît sous l'effet des fuites ou de l'absorption corporelle.
- de t0 à t0+$\Delta$t : l'appareil insuffle du gaz sous débit constant. La pression mesurée en tête de circuit augmente très rapidement jusqu'à se stabiliser à p0+$\Delta$p (au bout de 80 à 100 millisecondes pour les pressions et les circuits utilisés sur les appareils médicaux). Cette différence de pression $\Delta$p correspond à la perte de charge du circuit pour le débit et la configuration du circuit à l'instant t0. Cette perte de charge peut être mémorisée pour la durée du cycle d'insufflation afin de suivre la progression de la pression intracavitaire.
- de t0+$\Delta$t à t1 : l'appareil insuffle du gaz sous débit constant. L'écart entre la pression intracavitaire et la pression d'insufflation reste constant et égal à $\Delta$p.
- de t1 à t1+$\Delta$t1 : l'insufflation est interrompue. La pression Pi mesurée chute très rapidement pour atteindre la pression statique ou intracavitaire.
- de t1+$\Delta$t1 à t2 : l'appareil est au repos. La pression Pi mesurée correspond à la pression intracavitaire.

En application de ce processus, l'invention a essentiellement pour objet un insufflateur médical à régulation automatique de débit gazeux, comprenant un circuit d'insufflation d'un gaz neutre dans une cavité opératoire, en relation avec une source de gaz sous pression, et sur ce circuit une vanne pilotée de régulation de débit, l'insufflateur étant caractérisé en ce qu'il comprend des moyens de mesure de la pression d'insufflation en tête du circuit d'insufflation, des moyens pour calculer en permanence la pression intercavitaire à partir de la pression d'insufflation, ceci par l'intermédiaire d'une évaluation de la perte de charge du circuit, des moyens de comparaison entre la pression intracavitaire calculée et une valeur de consigne de cette pression intracavitaire, et des moyens de pilotage de la vanne de régulation de débit en fonction du résultat de la comparaison avec la valeur de consigne précitée, afin de fournir en permanence un débit de gaz neutre minimal pour compenser strictement les fuites de gaz hors de la cavité opératoire.

On réalise ainsi un dispositif qui renvoie de façon permanente une pression intracavitaire calculée, très proche de la pression intracavitaire réelle (moins de 2 % d'écart), et qui, par la mise en oeuvre d'une vanne de régulation de débit, notamment une vanne proportionnelle, dont l'ouverture est pilotée électroniquement, maintient la pression intracavitaire à la valeur de consigne en réduisant les débits et pressions d'insufflation au strict nécessaire, ceci en compensant strictement les fuites de gaz, ce qui induit une réduction de la pression limite d'insufflation à un niveau compatible avec la sécurité des patients. En cas de fuites de gaz importantes, l'appareil compense momentanément ces pertes en adaptant les débits et pressions d'insufflation ainsi que la pression maximale d'insufflation (seuil de sécurité). Par ailleurs, le dispositif proposé s'adapte instantanément à toutes configurations pratiques de circuit (tubes, aiguilles d'insufflation ou trocards,...) et à toutes modifications des conditions d'utilisation (tube coudé ou pincé,...).

Plus particulièrement, l'insufflateur médical objet de l'invention est conçu pour fonctionner de façon cyclique, les cycles d'insufflation et de mesure étant pilotés par une horloge, les moyens électroniques de l'appareil étant prévus pour assurer successivement, à chaque cycle :

-   la mesure de la pression intercavitaire P0 à l'instant initial t0, en l'absence d'insufflation, égale à la pression statique en tête du circuit,
-   l'affichage de la pression intercavitaire P0,
-   le déclenchement de l'insufflation à l'instant t0 sous un débit restant constant durant le cycle considéré,
-   la mesure de la pression d'insufflation Pi à un instant t0+$\Delta$t, la durée $\Delta$t étant prédéterminée,
-   le calcul de la perte de charge $\Delta$p du circuit, évaluée comme la différence (Pi-P0) des deux pressions précédemment mesurées,
-   la mise en mémoire de la perte de charge $\Delta$p spécifique au cycle d'insufflation considéré,
-   la mesure de la pression dynamique d'insufflation Pi à un instant t postérieur à l'instant t0+$\Delta$t,
-   le calcul de la pression intercavitaire Pa à l'instant t, égale à la différence Pi-$\Delta$p,
-   l'affichage de la pression intercavitaire Pa ainsi déterminée, et sa comparaison avec la valeur de consigne.

Selon un autre aspect de l'invention, l'insufflateur comprend un ensemble de régulation pneumatique qui se compose en combinaison, d'amont en aval :

-   d'un moyen de détente primaire ramenant la pression du gaz à insuffler, à la sortie d'une source de gaz telle que bouteille de stockage de gaz liquéfié, à une basse pression calibrée,
-   d'un filtre,
-   d'un clapet de sécurité d'entrée,
-   d'un capillaire de détente secondaire permettant d'obtenir un régime laminaire, auquel est associé un capteur de pression différentielle pour la détermination du débit gazeux instantané,
-   d'une vanne proportionnelle dont l'ouverture est asservie électriquement par comparaison entre le débit instantané et un débit de consigne,
-   d'un clapet de sécurité de sortie, et
-   d'un circuit de sortie du gaz sur lequel sont disposés les moyens de mesure de la pression d'insufflation, utilisés comme expliqué plus haut pour calculer et réguler la pression intracavitaire.

L'ensemble de régulation pneumatique, ainsi constitué, présente l'avantage d'une très grande compacité. Il permet en outre une mesure du débit instantané, par mesure de la perte de charge entre les deux extrémités du capillaire, grâce à un capteur différentiel de pression comme précisé plus bas. Toutefois, ce dispositif réalisant une détente adiabatique du gaz provoque, s'il n'y est pas remédié, un refroidissement important du gaz surtout à grand débit, et l'insufflation de gaz trop froids peut provoquer chez le patient des incidents non négligeables, d'autant plus que durant l'anesthésie la température corporelle n'est plus correctement régulée. De plus, la mesure du débit étant déterminée par mesure de la perte de charge entre les extrémités du capillaire, il est nécessaire de maintenir celui-ci à température constante afin de réaliser une mesure précise. C'est pourquoi l'ensemble de régulation pneumatique est avantageusement complété par des moyens de maintien en température de l'ensemble de ses composants, assurant notamment le réchauffage du gaz parcourant le capillaire jusqu'à une température de l'ordre de celle du corps, soit en pratique une température comprise entre 35°C et 40°C.

Selon un mode de réalisation particulier de l'invention, le sous-ensemble comprenant le capillaire de détente et le capteur différentiel de pression est constitué à partir de deux éléments cylindriques coaxiaux, montés l'un dans l'autre, le capillaire étant constitué par un espace libre de section annulaire compris entre les deux éléments cylindriques, tandis que deux prises de pression sont prévues dans l'élément cylindrique extérieur, respectivement en amont et en aval de la zone formant capillaire, pour alimenter le capteur différentiel de pression à partir duquel est déterminé le débit instantané de gaz. Le clapet de sécurité d'entrée peut aussi être incorporé à ce sous-ensemble, notamment au centre de l'élément cylindrique intérieur.

Une telle disposition est particulièrement avantageuse pour créer une détente gazeuse, tout en déterminant le débit de gaz, en appliquant la loi connue selon laquelle l'écoulement d'un gaz dans un conduit de section constante provoque une chute de pression régulière, proportionnelle à la longueur du conduit et au carré de la vitesse du gaz. A température constante et à condition d'avoir un écoulement dit "laminaire" (nombre de Reynolds inférieur à 2000), on peut écrire la relation :

$$P2\text{-}P1 = \frac{\Lambda \times l \times \rho \ Vm^2}{h \times 2}$$

dans laquelle :

P1 = pression "amont"
P2 = pression "aval"
l = longueur du conduit
$\Lambda$ = coefficient de perte de charge volumique par unité de longueur
h = espace entre les deux parois du conduit
$\rho$ = masse volumique du gaz à température T°
Vm = vitesse moyenne d'écoulement

Sachant que le débit gazeux D s'exprime par D = S x Vm, où S désigne la section du conduit, on peut en déduire :

$$D = \sqrt{\frac{(P2\text{-}P1) \times 2S \times h}{\Lambda\rho}}$$

Dans le cas d'un conduit de section annulaire, délimité par deux parois cylindriques de diamètres respectifs $\Phi1$ et $\Phi2$, le débit D est donné par la formule suivante :

$$D = \sqrt{\frac{\pi}{2\Lambda} \times (P2\text{-}P1) \times (\Phi2^2 - \Phi1^2) \times (\Phi2\text{-}\Phi1)}$$

Comme indiqué plus haut, le débit d'insufflation reste constant durant chaque cycle. Toutefois, ce débit varie, par sauts, d'un cycle à l'autre, pour permettre par paliers successifs (ouverture ou fermeture progressive de la vanne de régulation de débit) de compenser strictement les fuites de gaz afin de maintenir ou d'atteindre la pression intercavitaire de consigne.

L'aptitude du dispositif à compenser les fuites hors de la cavité opératoire est matérialisée par la variation de la fonction

$$\delta = \delta(P \text{ consigne - } P \text{ intracavitaire})/\delta t,$$

dérivée de la fonction $\Delta P = (P \text{ consigne-} P \text{ intracavitaire})$

Le paramétrage du débit doit répondre aux critères suivants :

- Plus l'écart $\Delta P$ entre la pression de consigne et la pression intracavitaire sera important, plus le saut de débit entre deux cycles devra être important, ceci afin d'atteindre rapidement la pression de consigne.
- Les sauts de débit seront négatifs si le débit d'insufflation est supérieur aux fuites, c'est-à-dire si $\delta$ est négatif ; ils seront positifs si $\delta$ est positif.
- Le débit devra être légèrement supérieur à la fuite de gaz constatée en fonctionnement stabilisé.
- Il ne doit pas y avoir dépassement de la pression maximale d'insufflation (voir ci-dessous).

- Le débit doit pouvoir être, si nécessaire, limité à un débit maximum de consigne (par exemple 1l/min en début d'intervention), noté ci-après D consigne.

Dans la suite, on précise le paramétrage du débit dans le cadre d'un exemple, avec référence aux figures 2 et 3 du dessin annexé, $\Delta$D désignant un saut de débit entre le débit D1 d'un premier cycle, et le débit D2 d'un second cycle suivant immédiatement le premier.

La figure 2 illustre le paramétrage en fonction de l'écart $\Delta$P entre la pression de consigne et la pression statique ou intercavitaire (exprimée en mm Hg), le saut de débit correspondant $\Delta$D' étant exprimé en l/min. On a ici :

$$\Delta D' = (D2-D1) = 0,3 \text{ (P consigne - P intercavitaire)} - 0,6$$

$$\Delta D' = (D2-D1) = 0,3 \text{ (P consigne - P1)} - 0,6$$

La figure 3 illustre le paramétrage en fonction de l'écart $\delta$ (P consigne - P intracavitaire) exprimant l'aptitude à compenser les fuites, dans le même système d'unités. Le saut de débit correspondant $\Delta$D" est donné ici par :

$$\Delta D" = (D2-D1) = 1,25 \times \delta \text{(P consigne - P intracavitaire)} + 0,5$$

$$\Delta D" = (D2-D1) = 1,25 \times \text{(P consigne - P1 - P consigne + P0)} + 0,5$$

$$\Delta D" = (D2-D1) = 1,25 \times \text{(P0 - P1)} + 0,5$$

Le débit D2 est déterminé à partir du débit D1 et des sauts élémentaires D' et D", selon la relation générale :

$$D2 = D1 + \alpha \, (\Delta D' + \Delta D")$$

en respectant la condition :

$$0,1 \text{ l/min} < D2 < D \text{ consigne}$$

En choisissant $\alpha = 1$, et en tenant compte des relations précédentes :

$$\Delta D' = 0,3 \text{ (P consigne - P1)} - 0,6$$

$$\Delta D" = 1,25 \times \text{(P0 - P1)} + 0,5$$

On obtient finalement :

$$D2 = D1 + 0,3 \text{ P consigne} + 1,25 \text{ P0} - 1,583 \text{ P1} - 0,1$$

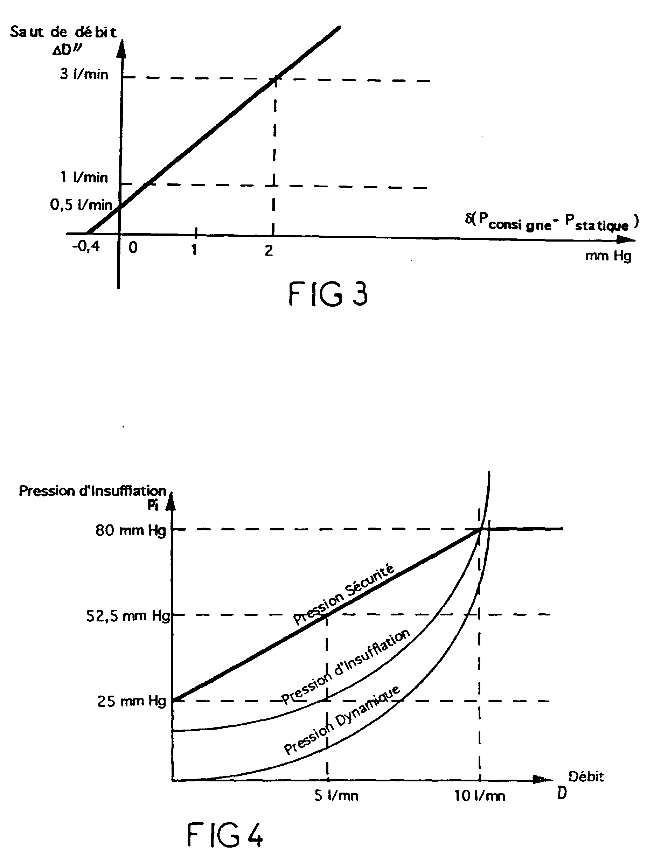

La pression d'insufflation, mesurée en aval de l'ensemble pneumatique comme indiqué ci-dessus, est de préférence volontairement limitée à un seuil maximum afin de réduire les risques d'accidents cardio-vasculaires pour le patient. Afin de tenir compte de la composante dynamique de la pression d'insufflation, ce seuil, appelé aussi pression de sécurité, est modulé en fonction du débit. Selon un mode de réalisation particulier, la pression de sécurité est modulée suivant une fonction linéaire croissante du débit, jusqu'à une valeur particulière du débit, et au-dessus de cette valeur du débit, ladite pression de sécurité est constante de manière à fixer une limite absolue à la pression d'insufflation. Le diagramme de la figure 4 illustre, dans le cas d'un exemple, une telle loi de variation de la pression de sécurité.

Afin de tenir compte des modifications pouvant intervenir à tout moment dans la configuration du circuit d'insufflation (coude sur le tube d'insufflation, déplacement de l'aiguille d'insufflation, etc...), l'appareil fonctionne selon un mode cyclique (cycle de 2 à 3 secondes par exemple), tous les paramètres tels que définis ci-dessus étant recalculés pour chaque cycle. Après chaque phase de repos, l'appareil redémarre au débit minimal.

Lorsque dans un cycle de progression des débits, la pression d'insufflation dépasse le seuil de sécurité ci-dessus discuté, l' insufflation s'arrête instantanément et l'appareil réduit le débit suivant la loi illustrée par la figure 4.

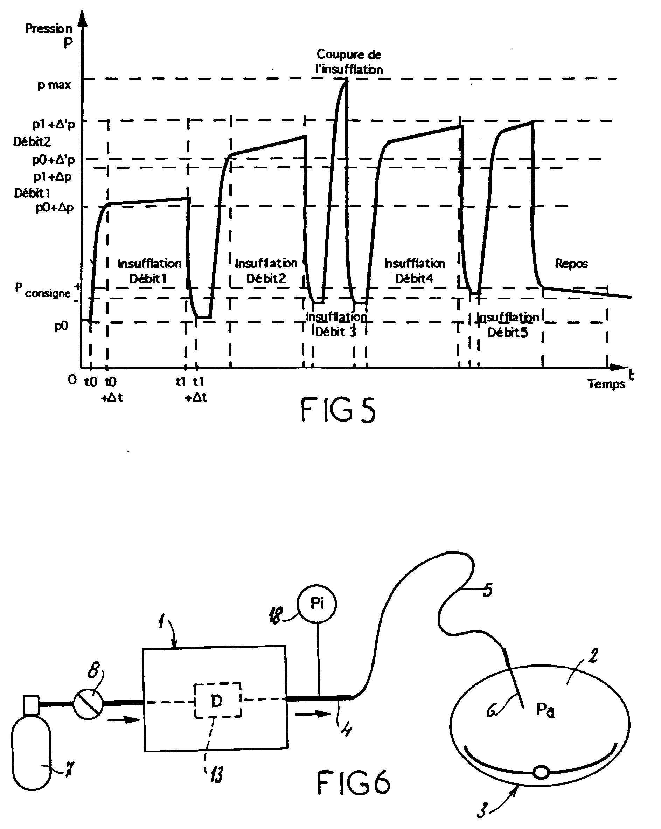

L'insufflateur s'arrête lorsque la pression intracavitaire calculée est égale à la pression de consigne. La figure 5 est un diagramme des pressions durant un cycle de montée des débits, illustrant les temps d'insufflation successifs avec des débits distincts, jusqu'à ce que soit atteinte la pression de consigne, une coupure de l'insufflation étant réalisée à un instant intermédiaire où la pression de sécurité pmax est atteinte.

L'invention sera mieux comprise à l'aide de la description qui suit, en référence au dessin schématique annexé représentant, à titre d'exemple non limitatif, une forme de réalisation de cet insufflateur médical à régulation automatique de débit gazeux :

Figure 6 est un schéma de principe montrant un insufflateur médical et son environnement ;

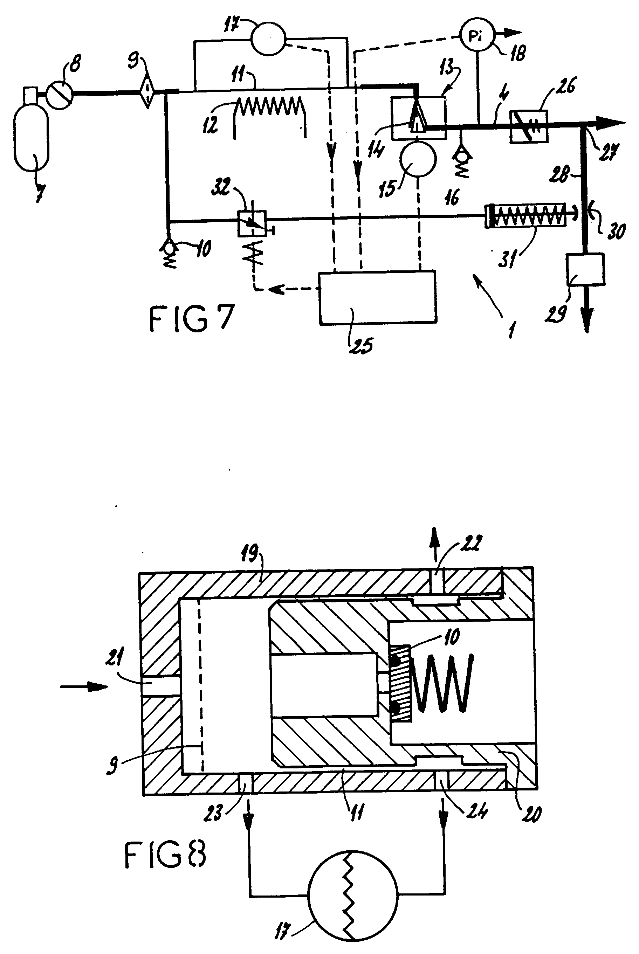

Figure 7 est un schéma synoptique de l'insufflateur médical conforme à la présente invention ;

Figure 8 est une vue de détail, en coupe, d'un mode d'exécution particulier du conduit capillaire auquel est associé le capteur différentiel de pression.

L'insufflateur médical, désigné globalement par le repère 1 sur la figure 6, assure l'insufflation d'un gaz neutre, tel que gaz carbonique CO2, dans une cavité opératoire 2 d'un patient 3. L'insufflation de gaz dans la cavité opératoire 2 s'effectue, depuis l'appareil 1, par l'intermédiaire d'un circuit comprenant une sortie 4 de gaz, un tube de liaison flexible 5 et une aiguille de Veress 6.

Le gaz est tenu en réserve, à pression élevée (par exemple 49 bars) et sous forme liquéfiée, dans une bouteille 7 à la sortie de laquelle est prévu un détendeur primaire 8 permettant de ramener la pression gazeuse à une basse pression calibrée et rigoureusement constante, par exemple de trois bars.

A l'intérieur du boîtier de l'appareil 1, comme le montre la figure 7, se trouve un étage de détente secondaire composé, d'amont en aval :

- d'un filtre 9, par exemple d'une porosité de 5 $\mu$,
- d'un clapet de sécurité d'entrée 10, taré par exemple à une pression de 3,5 bars,
- d'un capillaire de détente 11, maintenu à une température constante par un organe de chauffage 12, et permettant d'obtenir un régime laminaire,
- d'une vanne motorisée de régulation de débit 13, à action proportionnelle, comprenant un gicleur 14 actionné par un moteur 15, dont l'ouverture est asservie électroniquement,
- d'un clapet de sécurité de sortie 16, taré par exemple à une pression de 0,2 bar,
- de la sortie 4 vers le circuit d'insufflation du gaz dans la cavité opératoire 2.

Un capteur de pression différentielle 17 est associé au capillaire de détente. Un autre capteur de pression 18, disposé sur la sortie 4 vers le circuit d'insufflation (voir aussi figure 6), permet de déterminer la pression d'insufflation Pi par mesure directe. La pression intracavitaire Pa est déduite, par calcul, de la mesure de la pression d'insufflation Pi et de l'évaluation de la perte de charge dans le circuit d'insufflation.

En se référant à la figure 8, on décrira maintenant un mode d'exécution avantageux du capillaire de détente 11, réalisé à partir de deux éléments cylindriques 19 et 20, disposés coaxialement l'un dans l'autre. Le premier élément cylindrique 19, ou élément extérieur, comporte une entrée de gaz 21 à une extrémité, et il renferme le filtre 9. Le second élément cylindrique 20, ou élément intérieur, porte en son centre le clapet de sécurité d'entrée 10. Entre les deux éléments cylindriques 19 et 20 s'étend un espace annulaire, dont l'épaisseur (mesurée radialement) est par exemple de 0,16 mm, et qui constitue le conduit capillaire 11. La sortie de gaz s'effectue latéralement en 22, par un orifice percé dans la paroi du premier élément cylindrique 19, en aval du conduit capillaire 11 de section annulaire.

Le capteur différentiel de pression 17 utilise une première prise de pression latérale 23, sous la forme d'un orifice percé dans la paroi du premier élément cylindrique 19 en amont du conduit capillaire 11 de section annulaire, et une seconde prise de pression latérale 24, sous la forme d'un autre orifice percé dans la paroi du premier élément cylindrique 19 en aval du conduit capillaire 11.

Les deux capteurs de pression 17 et 18 sont reliés à un bloc électronique de mesure et de commande 25, qui pilote la vanne de régulation de débit 13 selon les procédures d'asservissement décrites plus haut, notamment pour maintenir la pression intracavitaire Pa à la valeur de consigne, tout en limitant la pression d'insufflation Pi à la valeur de seuil de sécurité voulue.

Un clapet anti-retour 26, équipant le départ du circuit d'insufflation évite toute pollution de l'appareil par des remontées de liquides ou de fumées, alors que la compensation des surpressions par aspiration sur le même tube de liaison 5 que celui destiné à l'insufflation du gaz dans la cavité opératoire 2 est en soi une cause certaine de pollution. Ce clapet anti-retour 26 est associé à un raccord en Y 27 situé au départ du tube de liaison 5, et servant au branchement d'un tube de dérivation 28 avec filtre à fumées 29, assurant l'échappement des surpressions vers l'extérieur tout en empêchant le rejet de fumées nocives (laser par exemple) dans l'atmosphère du bloc opératoire. Un dispositif 30 du genre vanne, tel qu'un dispositif dit à écrasement ou pincement de tube, actionné par un petit vérin pneumatique 31, permet le contrôle de l'aspiration et de l'échappement par le tube 28. Pour garantir la stérilité de cet ensemble, les tubes 5 et 28 et le filtre à fumées 29 sont avantageusement du type à usage unique.

Le vérin pneumatique 31 du dispositif à écrasement ou pincement de tube 30 peut être commandé soit de façon automatique en cas de détection d'une surpression, par l'intermédiaire d'une électrovanne d'échappement 32 pilotée par la pression intracavitaire calculée, soit de façon manuelle afin de permettre l'évacuation des fumées. Durant l'ouverture du dispositif 30, aucune mesure de pression n'est effectuée car le capteur 18 ne pourrait mesurer que la pression de l'échappement, proche de la pression ambiante. Ce dispositif ne doit agir que pour des surpressions durables ; son mode d'action automatique est donc d'une part temporisé, afin de ne pas prendre en compte certains effets sporadiques dus par exemple à l'introduction d'instruments par le chirurgien, et d'autre part limité aux pressions intracavitaires égales ou supérieures à la pression de consigne augmentée d'une valeur prédéterminée.

Alors que le fonctionnement décrit jusqu'à présent concerne le processus normal d'insufflation, il convient d'ajouter que l'appareil objet de l'invention permet aussi d'exécuter un cycle particulier de contrôle de la bonne implantation de l'aiguille, avant insufflation. A ce sujet, il est rappelé que les praticiens de l'endoscopie ont pour habitude d'injecter, avant insufflation, un faible volume d'air (de l'ordre de 20 cm$^3$) à l'aide d'une seringue au travers de l'aiguille d'insufflation, afin de s'assurer que celle-ci ne débouche pas dans un vaisseau sanguin ou entre les feuillets du péritoine. La pression intra-abdominale se situant normalement un peu en-dessous de la pression atmosphérique ambiante, l'injection de 20 cm$^3$ d'air dans une cavité représentant plus de 10 dm$^3$ ne provoque pas de changement significatif de la pression intra-abdominale ; cet effet est complété par l'absorption du gaz par l'ensemble de la surface péritonéale. Si l'aiguille n'a pas atteint la cavité abdominale, si elle se trouve implantée entre deux feuillets péritonéaux ou adhérentiels, ou piquée dans une anse intestinale, l'injection de 20 cm$^3$ d'air va entraîner une variation de la pression gazeuse dans la cavité ou a lieu l'injection, et le piston de la seringue permettra une certaine réaspiration "élastique". Grâce à l'appareil objet de la présente invention, ce test manuel est remplacé par un test autorisant ou non la mise en fonction de l'insufflateur. Une commande particulière de l'appareil déclenche automatiquement l'injection d'une quantité prédéterminée et faible, par exemple de 20 cm$^3$ de gaz au débit minimum soit 1l/min. Une mesure de la pression statique (intraabdominale) est effectuée avant l'injection. Une seconde mesure est effectuée après cette injection. Si les deux mesures diffèrent d'un écart supérieur à un certain seuil de tolérance, l'appareil est remis en attente, et il émet un signal d'alarme.

Enfin on notera que l'organe de chauffage 12, tel que résistance électrique de chauffage, permet non seulement de maintenir le gaz à température constante dans le capillaire 11 pour la mesure du débit gazeux par l'intermédiaire du capteur différentiel de pression 17, mais assure aussi le réchauffage de ce gaz (refroidi par la détente) jusqu'à une température compatible avec celle du corps, soit comprise entre 35°C et 40°C, et de préférence entre 38°C et 40°C, avant insufflation dans la cavité abdominale ou autre cavité opératoire.

## Revendications

1.  Insufflateur médical (1) à régulation automatique de débit gazeux, pour endoscopie diagnostique et chirurgicale, comprenant un circuit d'insufflation (4, 5, 6) d'un gaz neutre dans une cavité opératoire (2), en relation avec une source de gaz neutre sous pression (7), sur ce circuit une vanne pilotée de régulation de débit (13), et des moyens de mesure (18) de la pression d'insufflation (Pi) en tête du circuit d'insufflation (4,5,6), caractérisé en ce qu'il comprend des moyens (25) pour calculer en permanence la pression intercavitaire (Pa) à partir de la pression d'insufflation (Pi), ceci par l'intermédiaire d'une évaluation de la perte de charge (Δp) du circuit, des moyens de comparaison entre la pression intracavitaire (Pa) calculée et une valeur de consigne de cette pression intracavitaire, et des moyens de pilotage de la vanne de régulation de débit (13) en fonction du résultat de la comparaison avec la valeur de consigne précitée, afin de fournir en permanence un débit de gaz neutre (D) minimal pour compenser strictement les fuites de gaz hors de la cavité opératoire (2).

2.  Insufflateur médical à régulation automatique de débit gazeux selon la revendication 1, caractérisé en ce que ses moyens électroniques (25), pilotés par une horloge pour un fonctionnement cyclique, sont prévus pour assurer successivement, à chaque cycle :

    -   la mesure de la pression intercavitaire (P0) à un instant initial (t0), en l'absence d'insufflation, égale à la pression statique en tête du circuit d'insufflation (4,5,6),
    -   l'affichage de la pression intercavitaire (P0),
    -   le déclenchement de l'insufflation à l'instant (t0) sous un débit (D) restant constant durant le cycle considéré,
    -   la mesure de la pression d'insufflation (Pi) à un instant (t0+Δt), la durée (Δt) étant prédéterminée,
    -   le calcul de la perte de charge (Δp) du circuit, évaluée comme la différence (Pi-P0) des deux pressions précédemment mesurées,
    -   la mise en mémoire de la perte de charge (Δp) spécifique au cycle d'insufflation considéré,
    -   la mesure de la pression dynamique d'insufflation (Pi) à un instant (t) postérieur à l'instant (t0+Δt),
    -   le calcul de la pression intercavitaire (Pa) à l'instant (t), égale à la différence (Pi-Δp),
    -   l'affichage de la pression intercavitaire (Pa) ainsi déterminée, et sa comparaison avec la valeur de consigne.

3.  Insufflateur médical à régulation automatique de débit selon la revendication 2, caractérisé en ce qu'il comporte un moyen d'évaluation de l'aptitude de l'appareil à compenser les fuites hors de la cavité opératoire (2), par la dérivée (δ) par rapport au temps (t) de l'écart (ΔP) entre la pression de consigne et la pression intracavitaire (Pa).

4. Insufflateur médical à régulation automatique de débit gazeux selon l'une quelconque des revendications 1 à 3, caractérisé en ce qu'il comprend un ensemble de régulation pneumatique qui se compose en combinaison, d'amont en aval :

   - d'un moyen de détente primaire (8) ramenant la pression du gaz à insuffler, à la sortie d'une source de gaz telle que bouteille de stockage de gaz liquéfié (7), à une basse pression calibrée,
   - d'un filtre (9),
   - d'un clapet de sécurité d'entrée (10),
   - d'un capillaire de détente secondaire (11) permettant d'obtenir un régime laminaire, auquel est associé un capteur de pression différentielle (17) pour la détermination du débit gazeux instantané (D),
   - d'une vanne proportionnelle (13) dont l'ouverture est aservie électriquement par comparaison entre le débit instantané (D) et un débit de consigne (Dconsigne),
   - d'un clapet de sécurité de sortie (16), et
   - d'un circuit de sortie du gaz (4) sur lequel sont disposés les moyens de mesure (18) de la pression d'insufflation (Pi), utilisés pour calculer et réguler la pression intracavitaire (Pa).

5. Insufflateur médical à régulation automatique de débit gazeux selon la revendication 4, caractérisé en ce que l'ensemble de régulation pneumatique est complété par des moyens de maintien en température (12), assurant notamment le réchauffage du gaz parcourant le capillaire (11).

6. Insufflateur médical à régulation automatique de débit gazeux selon la revendication 4 ou 5, caractérisé en ce que le sous-ensemble comprenant le capillaire de détente (11) et le capteur différentiel de pression (17) est constitué à partir des deux éléments cylindriques (19,20) coaxiaux, montés l'un dans l'autre, le capillaire (11) étant constitué par un espace libre de section annulaire compris entre les deux éléments cylindriques (19,20), tandis que deux prises de pression (23,24) sont prévues dans l'élément cylindrique extérieur (19), respectivement en amont et en aval de la zone formant capillaire (11), pour alimenter le capteur différentiel de pression (17) à partir duquel est déterminé le débit instantané de gaz (D).

7. Insufflateur médical à régulation automatique de débit gazeux selon la revendication 6, caractérisé en ce que le clapet de sécurité d'entrée (10) est incorporé au sous-ensemble précité comprenant le capillaire de détente (11), notamment au centre de l'élément cylindrique intérieur (20).

8. Insufflateur médical à régulation automatique de débit gazeux selon l'une quelconque des revendications 1 à 7, caractérisé en ce qu'il comprend des moyens de définition et de modulation d'un seuil maximum de la pression d'insufflation (Pi), dit "pression de sécurité", notamment suivant une fonction linéaire croissante du débit gazeux (D), toutefois avec maintien de cette pression de sécurité à une valeur constante pour les débits se situant au-dessus d'une valeur particulière de débit, l'insufflation étant arrêtée notamment dans un cycle de progression des débits lorsque la pression d'insufflation (Pi) dépasse ladite pression de sécurité.

9. Insufflateur médical à régulation automatique de débit gazeux selon l'une quelconque des revendications 1 à 8, caractérisé en ce que le départ du circuit d'insufflation (4,5,6) est équipé d'un clapet antiretour (26), auquel est associé un raccord en Y (27) pour le branchement d'un tube de dérivation (28) avec filtre à fumées (29), assurant l'échappement vers l'extérieur des surpressions intracavitaires, un dispositif du genre vanne (30), tel qu'à écrasement ou pincement de tube assurant le contrôle de l'aspiration et de l'échappement par le tube (28), de façon automatique ou manuelle, la mesure de pression étant neutralisée durant l'ouverture dudit dispositif (30).

10. Insufflateur médical à régulation automatique de débit selon l'une quelconque des revendications 1 à 9, caractérisé en ce qu'il est prévu pour exécuter, outre le processus d'insufflation avec régulation du débit, un cycle particulier de contrôle avant insufflation, avec injection automatique d'une quantité prédéterminée et faible de gaz à un débit minimum, et mesures de pressions avant et après cette injection de gaz.

## Claims

1. Medical insufflator (1), with automatic regulation of the gas flow rate, for diagnostic and surgical endoscopy, comprising a circuit (4,5,6) for insufflation of a neutral gas into an operative cavity (2), relative to a pressurised neutral gas source (7), and on this circuit a piloted valve (13) for regulation of the flow rate, means (18) for measuring the insufflation pressure (Pi) at the head of the insufflation circuit (4,5,6), characterised in that it comprises means (25) for continuous calculation of the inter-cavity pressure (Pa) from the insufflation pressure (Pi), by means of evaluation of the loss of load (Δp) of the circuit, means for comparison of the intracavity pressure (Pa) calculated, and a required value of this intracavity pressure, and means for piloting the flow rate regulation valve (13), according to the result of the comparison with the aforementioned required value, in order to supply continuously a minimal neutral gas flow rate (D), such as to compensate accurately for leakages of gas outside the operative cavity (2).

2. Medical insufflator (1) according to claim 1, with automatic regulation of the gas flow rate, characterised in that its electronic means (25), which are piloted by a clock for cyclical operation, are provided in order to assure in succession in each cycle:

- measurement of the intercavity pressure (PO) at an initial moment (tO), in the absence of insufflation, which is equivalent to the static pressure at the head of the insufflation circuit (4,5,6);
- display of the intercavity pressure (PO);
- triggering of the insufflation at the moment (tO), with a flow rate (D) which remains constant during the cycle in question;
- measurement of the insufflation pressure (Pi) at a moment (tO+Δt), the duration (Δt) being pre-determined;
- calculation of the loss of load (Δp) of the circuit, evaluated as the difference (Pi-PO) of the two pressures previously measured;
- storage in the memory of the loss of load (Δp) which is specific to the insufflation cycle in question;
- measurement of the dynamic pressure of insufflation (Pi) at a moment (t) which is after the moment (tO+Δt);
- calculation of the intercavity pressure (Pa) at the moment (t), which is equivalent to the difference (Pi-Δp); and
- display of the intercavity pressure (Pa) thus determined, and comparison of this pressure with the required value.

3. Medical insufflator according to claim 2, with automatic regulation of the gas flow rate, characterised in that it comprises a means for evaluation of the capacity of the device to compensate for leakages outside the operative cavity (2), by means of the drift (δ) relative to the time (t) of the difference (ΔP) between the required pressure and the intracavity pressure (Pa).

4. Medical insufflator according to any one of claims 1 to 3, with automatic regulation of the gas flow rate, characterised in that it comprises a pneumatic regulation assembly, which consists of the following in combination, going from upstream towards downstream:

- a primary pressure reduction means (8), which reduces to a calibrated low pressure the pressure of the gas to be insufflated, at the outlet of a gas source such as a liquid gas storage cylinder (7);
- a filter (9);
- an intake safety flap valve (10);
- a secondary pressure reduction capillary unit (11), which makes it possible to obtain a laminar flow, with which there is associated a differential pressure sensor (17) for determination of the instantaneous gas flow rate (D);
- a proportional valve (13), opening of which is controlled electrically by comparison between the instantaneous flow rate (D) and a required flow rate (Dconsigne);
- an outlet safety flap valve (16); and
- a gas outlet circuit (4), on which there are disposed the means (18) for measuring the insufflation pressure (Pi), used in order to calculate and regulate the intracavity pressure (Pa).

5. Medical insufflator according to claim 4, with automatic regulation of the gas flow rate, characterised in that the pneumatic regulation assembly is completed by temperature maintenance means (12), which in particular assure re-heating of the gas which passes through the capillary unit (11).

6. Medical insufflator according to claim 4 or claim 5, with automatic regulation of the gas flow rate, characterised in that the sub-assembly which comprises the pressure reduction capillary unit (11) and the differential pressure sensor (17) consists of two, coaxial cylindrical components (19,20), mounted one in the other, the capillary unit (11) consisting of a free space with an annular cross-section, which is contained between the two cylindrical components (19,20), whereas two pressure sockets (23,24) are provided in the outer cylindrical component (19), respectively upstream and downstream from the area which forms the capillary unit (11), in order to supply to the differential pressure sensor (17), by means of which the instantaneous gas flow rate (D) is determined.

7. Medical insufflator according to claim 6, with automatic regulation of the gas flow rate, characterised in that the intake safety flap valve (10) is incorporated in the aforementioned sub-assembly which comprises the pressure reduction capillary unit (11), in particular in the centre of the inner cylindrical component (20).

8. Medical insufflator according to any one of claims 1 to 7, with automatic regulation of the gas flow rate, characterised in that it comprises means for definition and modulation of a maximum insufflation pressure threshold (Pi), which is known as the "safety pressure", in particular according to an increasing linear function of the gaseous flow rate (D), but with maintenance of this safety pressure at a constant value for flow rates which are higher than a specific flow rate value, the insufflation being stopped in particular in a flow-rate progression cycle, when the insufflation pressure (Pi) exceeds the said safety pressure.

9. Medical insufflator according to any one of claims 1 to 8, with automatic regulation of the gas flow rate, characterised in that the initial part of the insufflation circuit (4,5,6) is equipped with a non-return flap valve (26), with which there is associated a Y-shaped connection (27), for branching of a branching tube (28) with a fume filter (29), which provides release to the exterior of the intracavity excess pressures, and a device of the valve type (30), such that by means of compression or pinching of the tube, suction and release via the tube (28) is controlled automatically or manually, the pressure measurement being neutralised during opening of the said device (30).

10. Medical insufflator according to any one of claims 1 to 9, with automatic regulation of the flow rate, characterised in that as well as executing the insufflation process, with regulation of the flow rate, it is designed to execute a specific control cycle before insufflation takes place, with automatic injection of a small, predetermined quantity of gas at a minimum flow rate, and pressure measurements before and after this injection of gas.

## Patentansprüche

1. Medizinischer Gasinhalator (1) mit automatischer Gasflußregelung für diagnostische und chirurgische Endoskopie, umfassend einen Kreislauf (4, 5, 6) zum Inhalieren eines neutralen Gases in eine Operationshöhle (2) in Verbindung mit einer Quelle des unter Druck befindlichen neutralen Gases (7), in diesem Kreislauf ein gesteuertes Durchflußregelungsventil (13), und Mittel (18) zur Messung des Inhalationsdrucks (Pi) am Kopf des Inhalationskreislaufs (4, 5, 6),
dadurch gekennzeichnet, daß er umfaßt: Mittel (25), um ständig den interkavitären Druck (Pa) ausgehend vom Inhalationsdruck (Pi) zu berechnen, und zwar durch eine Bestimmung des Druckverlustes ($\Delta$p) des Kreislaufs, Mittel zum Vergleich zwischen dem berechneten intrakavitären Druck (Pa) und einem Sollwert dieses intrakavitären Drucks, und Mittel zur Steuerung des Durchflußregelungsventils (13) in Abhängigkeit vom Ergebnis des Vergleichs mit dem obengenannten Sollwert, damit ständig ein minimaler Durchfluß (D) des neutralen Gases geliefert wird, um Leckagen von Gas aus der Operationshöhle (2) exakt zu kompensieren.

2. Medizinischer Gasinhalator mit automatischer Gasflußregelung nach Anspruch 1, dadurch gekennzeichnet, daß seine elektronischen Mittel (25), die durch einen Taktgeber für einen zyklischen Betrieb gesteuert werden, vorgesehen sind, um nacheinander bei jedem Zyklus folgendes zu gewährleisten:

- die Messung des interkavitären Drucks (P0) zu einem Anfangszeitpunkt (t0) bei fehlender Inhalation, der gleich ist dem statischen Druck am Kopf des Inhalationskreislaufs (4, 5, 6),
- die Anzeige des interkavitären Drucks (P0),
- das Auslösen der Inhalation zum Zeitpunkt (t0) unter einem Durchfluß (D), der während des betreffenden Zyklus konstant bleibt,
- die Messung des Inhalationsdrucks (Pi) zu einem Zeitpunkt (t0 + $\Delta$t), wobei die Dauer ($\Delta$t) vorbestimmt ist,
- die Berechnung des Druckverlusts ($\Delta$p) des Kreislaufs, bestimmt als die Differenz (Pi - P0) der zwei vorher gemessenen Drücke,
- die Speicherung des für den betreffenden Inhalationszyklus spezifischen Druckverlusts ($\Delta$p),
- die Messung des dynamischen Inhalationsdrucks (Pi) zu einem Zeitpunkt (t) nach dem Zeitpunkt (t0 + $\Delta$t),
- die Berechnung des interkavitären Drucks (Pa) zum Zeitpunkt (t), der gleich ist der Differenz (Pi - $\Delta$p),
- die Anzeige des auf diese Weise bestimmten interkavitären Drucks (Pa), und seinen Vergleich mit dem Sollwert.

3. Medizinischer Gasinhalator mit automatischer Gasflußregelung nach Anspruch 2, dadurch gekennzeichnet, daß er ein Mittel zur Bestimmung der Fähigkeit des Apparates, die Leckagen aus der Operationshöhle (2) durch die Ableitung ($\delta$) nach der Zeit (t) der Differenz ($\Delta$P) zwischen dem Solldruck und dem intrakavitären Druck (Pa) zu kompensieren, umfaßt.

4. Medizinischer Gasinhalator mit automatischer Gasflußregelung nach einem der Ansprüche 1 bis 3, dadurch gekennzeichnet, daß er eine pneumatische Regelungseinheit umfaßt, die sich in Kombination von stromaufwärts nach stromabwärts zusammensetzt aus:

- einem Mittel zur primären Entspannung (8), das den Druck des zu inhalierenden Gases am Ausgang einer Gasquelle, wie z.B. einer Flasche zur Lagerung von Flüssiggas (7) auf einen niedrigen kalibrierten Druck bringt,
- einem Filter (9),
- einer Eingangs-Sicherheitsklappe (10),
- einer Kapillare zur sekundären Entspannung (11), die es ermöglicht, einen laminaren Strömungsbereich zu erhalten, mit dem ein Differentialdruckfühler (17) zur Bestimmung des momentanen Gasdurchflusses (D) verbunden ist,
- einem proportionalen Ventil (13), dessen Öffnung elektrisch durch den Vergleich zwischen dem momentanen Durchfluß (D) und einem Solldurchfluß (Dconsigne) geregelt wird,
- einer Ausgangs-Sicherheitsklappe (16), und
- einem Gasauslaßkreislauf (4), in dem die Mittel (18) zur Messung des Inhalationsdrucks (Pi) angeordnet sind, die zur Berechnung und Regelung des intrakavitären Drucks (Pa) verwendet werden.

5. Medizinischer Gasinhalator mit automatischer Gasflußregelung nach Anspruch 4, dadurch gekennzeichnet, daß die pneumatische Regelungseinheit ergänzt wird durch Mittel zur Aufrechterhaltung der Temperatur (12), die insbesondere für das Erwärmen des Gases sorgt, das die Kapillare (11) durchströmt.

6. Medizinischer Gasinhalator mit automatischer Gasflußregelung nach Anspruch 4 oder 5, dadurch gekennzeichnet, daß die Untereinheit, die die Entspannungskapillare (11) und den Differentialdruckfühler (17) umfaßt, auf der Basis von zwei koaxialen zylindrischen Elementen (19, 20), die ineinander montiert sind, gebildet wird, wobei die Kapillare (11) durch einen freien Raum mit ringförmigem Querschnitt zwischen den beiden zylindrischen Elementen (19, 20) gebildet wird, während zwei Druckfühler (23, 24) in dem äußeren zylindrischen Element (19) stromaufwärts bzw. stromabwärts von der Zone, die die Kapillare (11) bildet, vorgesehen sind, um den Differentialdruckfühler (17) zu speisen, auf dessen Basis der momentane Gasdurchfluß (D) bestimmt wird.

7. Medizinischer Gasinhalator mit automatischer Gasflußregelung nach Anspruch 6, dadurch gekennzeichnet, daß die Eingangs-Sicherheitsklappe (10) in die obengenannte, die Enspannungskapillare (11) umfassende Untereinheit integriert ist, insbesondere in der Mitte des inneren zylindrischen Elements (20).

8. Medizinischer Gasinhalator mit automatischer Gasflußregelung nach einem der Ansprüche 1 bis 7, dadurch gekennzeichnet, daß er Mittel zur Definition und Modulation eines oberen Grenzwerts für den Inhalationsdruck (Pi), „Sicherheitsdruck" genannt, umfaßt, insbesondere entsprechend einer steigenden linearen Funktion des Gasdurchflusses (D), jedoch mit Halten dieses Sicherheitsdrucks auf einem konstanten Wert bei Durchflüssen über einem bestimmten Durchflußwert, wobei die Inhalation insbesondere in einem Zyklus der Progression der Durchflüsse gestoppt wird, wenn der Inhalationsdruck (Pi) den genannten Sicherheitsdruck übersteigt.

9. Medizinischer Gasinhalator mit automatischer Gasflußregelung nach einem der Ansprüche 1 bis 8, dadurch gekennzeichnet, daß der Beginn des Inhalationskreislaufs (4, 5, 6) mit einer Rückschlagklappe (26) ausgestattet ist, mit der ein Y-Verbindungsstück (27) zur Abzweigung eines Ableitungsrohrs (28) mit Rauchfilter (29) verbunden ist, das für das Entweichen von intrakavitären Überdrücken nach außen sorgt, wobei eine Vorrichtung vom Typ Ventil (30) mit Quetschung oder Umklammerung des Rohrs auf automatische oder manuelle Weise für die Kontrolle der Ansaugung und des Ausstoßes durch das Rohr (28) sorgt, wobei die Druckmessung während der Öffnung der genannten Vorrichtung (30) neutralisiert wird.

10. Medizinischer Gasinhalator mit automatischer Gasflußregelung nach einem der Ansprüche 1 bis 9, dadurch gekennzeichnet, daß er dazu vorgesehen ist, außer dem Inhalationsvorgang mit Durchflußregelung einen besonderen Kontrollzyklus vor dem Inhalieren mit automatischer Injektion einer vorbestimmten und geringen Gasmenge mit einem minimalen Durchfluß sowie Druckmessungen vor und nach jeder Gasinjektion durchzuführen.

Pression Pi

p1+Δp

p0+Δp

Repos | Insufflation mesure de la pression dynamique | Repos: mesure de la Pression statique ou Intracavitaire | Insufflation

p1

p0

O      t0   t0+Δt    t1   t1+Δt1          t2      Temps

$t$

## FIG 1

Saut de débit
ΔD'

3 l/min

1 l/min

$(P_{consigne} - P_{statique})$

0   2    5      10 12       20      mm Hg

-0,6 l/min

## FIG 2

Saut de débit
ΔD″

3 l/min

1 l/min
0,5 l/min

-0,4    0    1    2

$\delta(P_{consigne} - P_{statique})$

mm Hg

# FIG 3

Pression d'Insufflation
Pi

80 mm Hg

Pression Sécurité

52,5 mm Hg

Pression d'Insufflation

25 mm Hg

Pression Dynamique

Débit

5 l/mn       10 l/mn       D

# FIG 4

FIG 5

FIG 6

FIG 7

FIG 8